(19) 

Europäisches
Patentamt
European
Patent Office
Office européen
des brevets

(11) **EP 4 624 578 A1**

(12) **EUROPEAN PATENT APPLICATION**
published in accordance with Art. 153(4) EPC

(43) Date of publication:
 01.10.2025 Bulletin 2025/40

(21) Application number: 22966300.0

(22) Date of filing: 28.11.2022

(51) International Patent Classification (IPC):
 *C12N 15/62* (2006.01) *C07K 19/00* (2006.01)
 *A61K 39/00* (2006.01) *A61P 35/00* (2006.01)
 *A61P 37/02* (2006.01)

(52) Cooperative Patent Classification (CPC):
 A61K 39/00; A61P 35/00; A61P 37/02;
 C07K 19/00; C12N 15/62

(86) International application number:
 PCT/CN2022/134772

(87) International publication number:
 WO 2024/108614 (30.05.2024 Gazette 2024/22)

(84) Designated Contracting States:
 AL AT BE BG CH CY CZ DE DK EE ES FI FR GB
 GR HR HU IE IS IT LI LT LU LV MC ME MK MT NL
 NO PL PT RO RS SE SI SK SM TR
 Designated Extension States:
 BA
 Designated Validation States:
 KH MA MD TN

(30) Priority: 22.11.2022 CN 202211464658

(71) Applicant: Carbiogene Therapeutics Co., Ltd.
 Hangzhou, Zhejiang 310051 (CN)

(72) Inventors:
 • ZHU, Jiangao
  Hangzhou, Zhejiang 310051 (CN)

 • YANG, Xin
  Hangzhou, Zhejiang 310051 (CN)
 • YANG, Wenjun
  Hangzhou, Zhejiang 310051 (CN)

(74) Representative: Frick, Robert
 Lorenz Seidler Gossel
 Rechtsanwälte Patentanwälte
 Partnerschaft mbB
 Widenmayerstraße 23
 80538 München (DE)

Remarks:
 The complete document including Reference
 Table(s) and the Sequence Listing(s) can be
 downloaded from the EPO website

(54) **FUSION GENE CONTAINING ENCODING GENE OF CHIMERIC ANTIGEN RECEPTOR AND ENCODING GENE OF CHIMERIC SWITCH RECEPTOR AND USE THEREOF**

(57) A fusion gene containing an encoding gene of a chimeric antigen receptor and an encoding gene of a chimeric switch receptor and the use thereof. Specifically disclosed are a fusion protein with the amino acid sequence of SEQ ID NO. 1 and the nucleic acid molecule (SEQ ID NO. 3) comprising an encoding gene of a chimeric antigen receptor and an encoding gene of a chimeric switch receptor. By means of designing said fusion gene containing the encoding gene of the chimeric antigen receptor and the encoding gene of the chimeric switch receptor, PD-L1 activated inhibition of anti-tumor T cell responses is converted into a CD27-mediated activation signal, thereby endowing CAR-T cells with the capability of resisting an immunosuppressive tumor microenvironment, thereby enhancing its in vivo persistence. In addition, the micro-environment near a tumor is regulated by means of promoting secretion of cytokines IFN-$\gamma$ and IFN-$\alpha$2 so as to relieve the immunosuppression thereof, thereby mobilizing the organism auto-immunity to participate in the killing effect on tumor cells, and improving the anti-tumor effect of the CAR-T cells.

EP 4 624 578 A1

**Description**

**TECHNICAL FIELD**

The present invention relates to the field of biotechnology, and in particular relates to a fusion gene containing encoding gene of chimeric antigen receptor and encoding gene of chimeric switch receptor (CSR) and use thereof.

**BACKGROUND**

Chimeric antigen receptor (CAR) technology uses genetic engineering technology to modify immune cells to express exogenous anti-tumor genes, so that immune cells such as lymphocytes have the ability to recognize tumor cell surface antigens and specifically recognize and kill tumor cells. In recent years, CAR-T cell therapy has achieved encouraging results in hematological tumors such as B cells, but its great success in hematological tumors has not yet been replicated in solid tumors. Solid tumors are different from hematological tumors. When entering solid tumors, CAR-T cells need to overcome the inherent heterogeneity of tumor cells and the complexity of the inhibitory tumor microenvironment. The immunosuppression of the tumor microenvironment and the complexity of its structure are the main obstacles to the widespread application of T cell immunotherapy in solid tumors.

Specific recognition of tumor antigens is only the first step in the success of CAR-T. In order to survive, the tumor creates an immunosuppressive microenvironment for itself, which is unfriendly to CAR-T. Glycolysis of tumor cells makes the environment hypoxic, acidic, and nutrient-depleted. The execution of CAR-T functions also requires glycolysis and oxidative phosphorylation, but sugar is depleted by tumor cells, and the effector function of CAR-T cells is thus impaired. In an inflammatory environment, tumor cells usually upregulate inhibitory ligands such as PD-L1 and Galectin-9. In addition, the tumor microenvironment also has a large number of inhibitory immune cells and stromal cells, such as tumor-associated fibroblasts (CAFs), myeloid-derived suppressor cells (MDSCs), tumor-associated macrophages (TAMS), tumor-associated neutrophils (TANS), mast cells, and regulatory T cells (Tregs). VEGF, TGF-β, etc. secreted by these cells and tumor cells can cause tumor vascular malformations, promote anti-inflammatory polarization of immune cells such as TAMS, and participate in epithelial-mesenchymal transition (EMT). They can also produce reactive oxygen species (ROS) and molecules such as lactate, indoleamine 2,3-dioxygenase (Ido), prostaglandin e2 (PGE2), soluble fatty acids, and adenosine to form an inhibitory immune microenvironment. Recent studies have shown that the immunosuppressive effect of the tumor microenvironment becomes stronger after CAR-T cell therapy. In view of this, how to perform corresponding structural modification on CAR-T cells to overcome the immunosuppressive tumor microenvironment and thus improve the clinical treatment effect is still a severe challenge facing by CAR-T cell therapy. Researching and developing new CAR-T cells with powerful anti-tumor effects has important theoretical significance and application value for immune cell therapy of tumors.

**SUMMARY**

The technical problem to be solved by the present invention is how to prevent or treat tumors (such as tumors expressing GPC3 antigen) and/or regulate the immunosuppressive effect of the tumor microenvironment. The technical problem to be solved is not limited to the described technical subject matter, and those skilled in the art can clearly understand other technical subjects not mentioned in this article through the following description.

To solve the above technical problem, the present invention first provides a nucleic acid molecule, the nucleic acid molecule may include encoding gene of chimeric antigen receptor (CAR) and encoding gene of chimeric switch receptor (CSR).

Further, in the above nucleic acid molecule, the chimeric switch receptor may include a TGFβ receptor type II extracellular region (type II receptor extracellular region of transforming growth factor β), μPD-1 and CD27 transmembrane region and cytoplasmic region, and the μPD-1 may be a extracellular region of mutation-optimized PD-1, and the amino acid sequence of μPD-1 may be 187-332 of SEQ ID No.1. Further, in the above nucleic acid molecules, the encoding gene of the chimeric switch receptor may be any of the following:

B1) a nucleic acid molecule encoding a fusion protein;
B2) a DNA molecule whose encoding sequence is SEQ ID No.2;
B3) a DNA molecule whose nucleotide sequence is SEQ ID No.2;

The fusion protein may be any of the following:

A1) a protein whose amino acid sequence is SEQ ID No.1;
A2) a protein having 80% or more identity with the protein shown in A1) and having the same function obtained by replacing and/or deleting and/or adding amino acid residues of the amino acid sequence shown in SEQ ID No.1;
A3) a fusion protein having the same function obtained by attaching a tag to the N-terminus and/or C-terminus of A1) or A2).

The fusion protein may be a chimeric switch receptor (CSR).

The fusion protein and encoding gene thereof may be used to improve the ability of CAR cells to resist immunosuppressive tumor microenvironment, make CAR cells more persistent in vivo and/or inhibit the PD-1 and TGFβ signaling pathways to enhance the effect of CD27 signaling pathway.

The encoding gene of the fusion protein can be used to prepare the nucleic acid molecule of the present invention or the CAR cell containing the nucleic acid molecule of the present invention.

The tags described herein include but are not limited to: GST (glutathione sulfhydryl transferase) tagged protein, His6 tagged protein (His-tag), MBP (maltose binding protein) tagged protein, Flag tagged protein, SUMO tagged protein, HA tagged protein, Myc tagged protein, eGFP (enhanced green fluorescent protein), eCFP (enhanced cyan fluorescent protein), eYFP (enhanced yellow-green fluorescent protein), mCherry (monomeric red fluorescent protein) or AviTag tagged protein.

A person skilled in the art can easily mutate the nucleotide sequence encoding the fusion protein of the present invention by using known methods, such as directed evolution or point mutation. Those artificially modified nucleotides which having 75% or higher identity with the nucleotide sequence of the fusion protein of the present invention are derived from the nucleotide sequence of the present invention and are equivalent to the sequence of the present invention as long as they encode the fusion protein and have the function of the fusion protein.

The aforementioned 75% or more identity may be 80%, 85%, 90% or 95% or more identity.

Herein, identity refers to the identity of an amino acid sequence or a nucleotide sequence. The identity of an amino acid sequence can be determined using a homology search site on the Internet, such as the BLAST page on the NCBI homepage website. For example, in Advanced BLAST2.1, by using blastp as a program, setting the Expect value to 10, setting all filters to OFF, using BLOSUM62 as a Matrix, setting the Gap existence cost, Per residue gap cost, and Lambda ratio to 11, 1, and 0.85 (default values), respectively, and searching to calculate the identity of the amino acid sequence, the value of the identity (%) can be obtained.

Herein, the 80% or more identity can be at least 80%, 81%, 82%, 83%, 84%, 85%, 86%, 87%, 88%, 89%, 90%, 91%, 92%, 93%, 94%, 95%, 96%, 97%, 98%, or 99% identity.

Further, in the above-mentioned nucleic acid molecule, the nucleic acid molecule may further include IFNα gene (α interferon gene) and/or P2A gene.

The nucleotide sequence of the IFNα gene may be position 1552-2118 of SEQ ID No.3.

The nucleotide sequence of the P2A gene may be position 1474-1551 of SEQ ID No.3.

Further, in the nucleic acid molecule, the encoding gene of the chimeric antigen receptor may be an encoding gene of a chimeric antigen receptor targeting GPC3.

The GPC3 is phosphatidylinositol proteoglycan 3 (Glypican-3).

Further, the encoding gene of the chimeric antigen receptor targeting GPC3 may be any of the following:

C1) a nucleic acid molecule encoding a protein with an amino acid sequence of SEQ ID No.4;
C2) a DNA molecule having an encoding sequence of position 1-1473 of SEQ ID No.3;
C3) a DNA molecule having a nucleotide sequence of position 1-1473 of SEQ ID No.3.

Further, the nucleic acid molecule may be any of the following:

D1) a DNA molecule having a nucleotide sequence of SEQ ID No.3;
D2) a DNA molecule having 70% or more identity with the DNA molecule shown in D1) and having the same function obtained by modifying the nucleotide sequence and/or replacing and/or deleting and/or adding one or more nucleotides shown in SEQ ID No.3.

The 70% or more identity may be at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% identity.

The present invention also provides a biological material, which can be any of the following:

E1) an expression cassette containing the nucleic acid molecule;
E2) a recombinant vector containing the nucleic acid molecule, or a recombinant vector containing the expression cassette of E1);
E3) a recombinant microorganism containing the nucleic acid molecule, or a recombinant microorganism containing the expression cassette of E1), or a recombinant microorganism containing the recombinant vector of E2);

E4) a recombinant cell containing the nucleic acid molecule, or a recombinant cell containing the expression cassette of E1), or a recombinant cell containing the recombinant vector of E2);

E5) the encoding gene of the chimeric switch receptor;

E6) the chimeric switch receptor;

E7) the fusion protein.

In the above-mentioned biological materials, the cells in E4) can be T cells, NK cells, γδT cells, NKT cells, macrophages or stem cells.

The recombinant cells express the nucleic acid molecules.

Further, the cells in E4) can be T cells.

Further, the cells in E4) can be human T cells.

The vector described herein refers to a vector that can carry exogenous DNA or target genes into host cells for amplification and expression. The vector can be a cloning vector or an expression vector, including but not limited to: plasmids, phages (such as lambda phages or M13 filamentous phages, etc.), cosmids (i.e., cosmids), viral vectors (such as baculoviruses, retroviruses (including lentiviruses), adenoviruses, adeno-associated viruses or herpes viruses (such as herpes simplex viruses), etc.). In one or more embodiments of the present invention, the vector is a pUC57 vector and/or a retrovirus vector MP71.

The microorganism described herein can be bacteria, yeast, algae or fungi. Wherein, the bacteria may be from *Escherichia sp., Erwinia sp., Agrobacterium sp., Flavobacterium sp., Alcaligenes sp., Pseudomonas sp., Bacillus sp., etc., but not limited thereto. The yeast may be from Saccharomyces sp., Pichia sp., Yarrowia sp., Candida sp., Schizosaccharomyces sp., Hansenula sp., Kluyreromyces sp.,* etc., but not limited thereto. The algae may be from *Fucus sp., Achnanthes sp., Amphiprora sp., Amphora sp., Ankistrodesmus sp., Asteromonas sp., Boekelovia sp.,* etc., but not limited thereto. The fungi may be from *Fusarium sp., Rhizoctonia sp., Verticillium sp., Penicillium sp., Aspergillus sp., Cephalosporium sp.,* etc., but not limited thereto. In one or more embodiments of the present invention, the microorganism is *Escherichia coli* DH5α.

The cells (host cells) described herein refer to cells that can be used to introduce vectors, including but not limited to: mammalian immune cells or stem cells with the ability to differentiate into immune cells. The host cell may include T cells, NK cells, γδT cells, NKT cells, macrophages or stem cells, etc., but not limited thereto. In one or more embodiments of the present invention, the cell is a human T cell.

The recombinant vector described herein refers to a recombinant vector DNA molecule constructed by connecting an exogenous target gene with a vector in vitro, and can be constructed in any suitable manner, as long as the constructed recombinant vector can carry the exogenous target gene into the recipient cell and provide the exogenous target gene with replication, integration, amplification and/or expression ability in the recipient cell. In one or more embodiments of the present invention, the recombinant vector is a recombinant vector pUC57-GPC3 CAR-IC and/or a recombinant retroviral vector MP71-GPC3 CAR-IC.

The recombinant vector pUC57-GPC3 CAR-IC is a recombinant vector obtained by cloning the GPC3 CAR-IC gene with a nucleotide sequence of SEQ ID No.3 into the pUC57 vector. The recombinant vector pUC57-GPC3 CAR-IC contains the DNA molecule shown in SEQ ID No.3.

The recombinant retroviral vector MP71-GPC3 CAR-IC is a recombinant expression vector obtained by replacing the fragment (small fragment) between the *Not*I and *Eco*RI recognition sites of the retroviral vector MP71 with the DNA fragment of SEQ ID No.3 in the sequence list, while keeping the other nucleotide sequences of the retroviral vector MP71 unchanged. The recombinant retroviral vector MP71-GPC3 CAR-IC contains the DNA molecule shown in SEQ ID No.3.

The recombinant microorganism described herein refers to a microorganism with a changed function obtained by operating and modifying the genes of the target microorganism. For example, a recombinant microorganism obtained by introducing an exogenous target gene or a recombinant vector into the target microorganism, or a recombinant microorganism obtained by directly editing the endogenous gene of the target microorganism. In one or more embodiments of the present invention, the recombinant microorganism is a recombinant bacterium obtained by introducing the recombinant retroviral vector MP71-GPC3 CAR-IC into *Escherichia coli* DH5α, which contains the DNA molecule shown in SEQ ID No.3.

The recombinant cell described herein refers to a cell with a changed function obtained by operating and modifying the genes of the target cell. For example, a recombinant cell obtained by introducing an exogenous target gene or a recombinant vector into the target cell. Further, the recombinant cells described herein include recombinant cells obtained by stably expressing the CAR gene in receptor cells (such as T cells, NK cells, γδT cells, NKT cells, macrophages or stem cells), such as CAR-T cells, CAR-NK cells, CAR-macrophages (CAR-M cells), CAR-iPSC or CAR-PSC, but not limited thereto. In one or more embodiments of the present invention, the recombinant cell is a GPC3 CAR-IC T cell.

The GPC3 CAR-IC T cell is a recombinant cell obtained by packaging the recombinant retroviral vector MP71-GPC3 CAR-IC with a packaging cell to obtain a recombinant retrovirus, and then introducing the recombinant retrovirus into a T cell. The packaging cell can be a Phoenix Ecotropic (ECO) cell and a PG13 cell.

The GPC3 CAR-IC T cell contains the DNA molecule shown in SEQ ID No.3 and expresses the GPC3 CAR-IC gene (SEQ ID No.3).

The present invention also provides any of the following uses of any of the nucleic acid molecules described herein, and/or the biomaterials:

F1) use in the preparation of a drug for preventing or treating tumors;

F2) use in the preparation of a drug for preventing or treating tumors expressing the GPC3 antigen;

F3) use in regulating the immunosuppressive effect of the tumor microenvironment or in the preparation of a product regulating the immunosuppressive effect of the tumor microenvironment;

F4) use in the prevention or treatment of tumors;

F5) use in the prevention or treatment of tumors expressing the GPC3 antigen;

F6) use in the prevention or treatment of liver cancer, melanoma, Wilm's tumor, non-small cell lung cancer, ovarian clear cell carcinoma, squamous cell carcinoma, renal cell carcinoma, prostate cancer, colorectal cancer, hepatoblastoma or glioma.

Further, the regulation described in F3) may be reduction or inhibition.

In the above uses, the tumor expressing the GPC3 antigen may be liver cancer, melanoma, Wilm's tumor, non-small cell lung cancer, ovarian clear cell carcinoma, squamous cell carcinoma, renal cell carcinoma, prostate cancer, colorectal cancer, hepatoblastoma or glioma, but is not limited thereto.

Further, the liver cancer may be hepatocellular carcinoma (HCC).

Further, the squamous cell carcinoma may be lung squamous cell carcinoma.

Further, the melanoma may be malignant melanoma.

Further, the glioma may be glioblastoma.

Further, the tumor expressing the GPC3 antigen may be a tumor with high expression of GPC3.

Further, the F1) may be used in the preparation of a drug for inhibiting and/or killing a tumor.

Further, the F2) may be used in the preparation of a drug for inhibiting and/or killing a tumor expressing the GPC3 antigen.

The inhibition may be inhibition of tumor proliferation and/or growth.

The present invention also provides a pharmaceutical composition, wherein the active ingredient of the pharmaceutical composition may be a CAR cell (such as CAR-T cell, CAR-NK cell, CAR-macrophage, CAR-iPSC or CAR-PSC) containing or expressing any of the nucleic acid molecules of the present invention.

Further, the pharmaceutical composition includes the GPC3 CAR-IC T cells described herein.

Further, the pharmaceutical composition also includes one or more pharmaceutically acceptable carriers. The pharmaceutically acceptable carrier may be a diluent, an excipient, a filler, a binder, a wetting agent, a disintegrant, an absorption promoter, an adsorption carrier, a surfactant or a lubricant but is not limited thereto.

The pharmaceutical composition has an anti-tumor effect and can be used to prevent or treat tumors. Further, the pharmaceutical composition can be used to prevent or treat mammalian tumors.

The mammal may be any one of a human, a mouse, a rat, a guinea pig, a hamster, a pig, a dog, a sheep, a monkey, a rabbit, a cat, a cow, and a horse but is not limited thereto.

The tumor described herein may be a solid tumor (such as pancreatic cancer, lung cancer, kidney cancer, liver cancer or brain glioma).

Further, the active ingredient of the pharmaceutical composition may be an immune effector cell expressing the GPC3 CAR-IC gene (SEQ ID No.3).

Further, the active ingredient of the pharmaceutical composition may be the GPC3 CAR-IC T cell.

The present invention also provides a method for preventing or treating tumors, which may be administering a drug containing the recombinant cell described in E4) herein to a patient suffering from a tumor disease.

In the above method, the recombinant cell may be a GPC3 CAR-IC T cell, and the GPC3 CAR-IC T cell may contain a DNA molecule shown in SEQ ID No.3.

In the above method, the tumor may be a tumor expressing the GPC3 antigen.

In the above method, the tumor expressing the GPC3 antigen may be liver cancer, melanoma, Wilm's tumor, non-small cell lung cancer, ovarian clear cell carcinoma, squamous cell carcinoma, renal cell carcinoma, prostate cancer, colorectal cancer, hepatoblastoma or glioma, but is not limited thereto.

In the above method, the liver cancer may be hepatocellular carcinoma.

In the above method, the squamous cell carcinoma may be lung squamous cell carcinoma.

In the above method, the melanoma may be malignant melanoma.

In the above method, the glioma may be glioblastoma.

The present invention also provides a method for preparing the GPC3 CAR-IC T cells, the method comprising:

(1) constructing and synthesizing the GPC3 CAR-IC gene (SEQ ID No. 3);

(2) cloning the GPC3 CAR-IC gene into the retroviral vector MP71 to obtain a recombinant retroviral vector MP71-GPC3 CAR-IC;

(3) introducing the recombinant retroviral vector MP71-GPC3 CAR-IC into a packaging cell for packaging to obtain a recombinant retrovirus;

(4) transfecting the recombinant retrovirus into T cells to obtain the GPC3 CAR-IC T cells.

In the above method, the packaging cells may be Phoenix Ecotropic (ECO) cells and PG13 cells.

The present invention also provides the use of the fusion protein and/or the nucleic acid molecule in preparing GPC3 CAR-IC T cells.

The nucleic acid molecule described herein may be a fusion gene containing an encoding gene of the chimeric antigen receptor and an encoding gene of the chimeric switch receptor.

The nucleic acid molecule described herein can express a chimeric antigen receptor, IFNα and a fusion protein, and the fusion protein can be any of the following:

A1) a protein whose amino acid sequence is SEQ ID No.1;

A2) a protein having 80% or more identity and the same function as the protein shown in A1) obtained by replacing and/or deleting and/or adding amino acid residues of the amino acid sequence shown in SEQ ID No.1;

A3) a fusion protein having the same function obtained by attaching a tag at the N-terminus and/or C-terminus of A1) or A2).

Further, the target of the chimeric antigen receptor can be GPC3.

Further, the chimeric antigen receptor can be any of the following:

F1) a protein whose amino acid sequence is SEQ ID No.4;

F2) a protein having 80% or more identity and the same function as the protein shown in F1) obtained by replacing and/or deleting and/or adding amino acid residues of the amino acid sequence shown in SEQ ID No.4;

F3) a fusion protein having the same function obtained by attaching a tag at the N-terminus and/or C-terminus of F1) or F2).

Furthermore, the IFNα may be any of the following:

G1) a protein whose amino acid sequence is SEQ ID No.5;

G2) a protein having 80% or more identity with the protein shown in G1) and having the same function obtained by replacing and/or deleting and/or adding amino acid residues in the amino acid sequence shown in SEQ ID No.5;

G3) a fusion protein having the same function obtained by attaching a tag to the N-terminus and/or C-terminus of G1) or G2).

Further, the nucleic acid molecule described herein can express a chimeric antigen receptor with an amino acid sequence of SEQ ID No.4, an IFNα with an amino acid sequence of SEQ ID No.5, and a fusion protein (chimeric switch receptor) with an amino acid sequence of SEQ ID No.1.

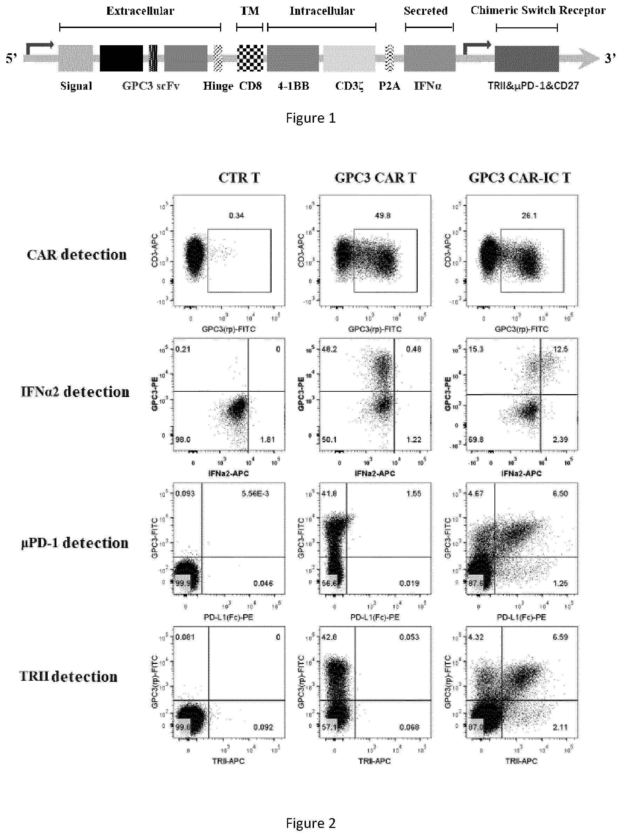

The nucleic acid molecule described herein can be a GPC3 CAR-IC gene, the nucleotide sequence of the GPC3 CAR-IC gene can be SEQ ID No.3, and the GPC3 CAR-IC gene can be an encoding gene for a chimeric antigen receptor, a P2A gene, an IFNα gene, a promoter element gene, and an encoding gene for a chimeric switch receptor from the N-terminus to the C-terminus. The structural schematic diagram is shown in Figure 1 (the three parts of the IFNα gene, the promoter element gene, and the encoding gene for the chimeric switch receptor can be collectively referred to as the IC gene). Wherein:

The nucleotide sequence of the IC gene (IFNα gene + promoter element gene + encoding gene for the chimeric switch receptor) can be 1552-3882 of SEQ ID No.3.

The encoding genes of the chimeric antigen receptor can be Signal (signal peptide, GPC3 scFv (tumor antigen binding region, i.e. single-chain antibody region), Hinge (hinge region), CD8 (transmembrane region), 4-1BB (intracellular signal region), CD3ζ (intracellular signal region) from N-terminus to C-terminus. The nucleotide sequence of the encoding gene of the chimeric antigen receptor can be positions 1-1473 of SEQ ID No.3, and the amino acid sequence encoding the chimeric antigen receptor (CAR) can be SEQ ID No.4.

In the encoding gene of chimeric antigen receptor, the nucleotide sequence of the encoding gene of Signal (signal peptide can be 1-63 of SEQ ID No.3, the nucleotide sequence of the encoding gene of GPC3 scFv can be 64-789 of SEQ ID No.3, the nucleotide sequence of the encoding gene of Hinge (hinge region) plus CD8 (transmembrane region) can be 790-996 of SEQ ID No.3, the nucleotide sequence of the encoding gene of 4-1BB (intracellular signal region) can be 997-1137 of SEQ ID No.3, and the nucleotide sequence of the encoding gene of CD3ζ (intracellular signal region) can be 1138-1473 of SEQ ID No.3.

The nucleotide sequence of the P2A gene can be 1474-1551 of SEQ ID No.3. The P2A peptide encoded by it can be a short peptide derived from a virus, which is usually called a "self-cleavage" peptide. The "self-cleavage" function of the P2A peptide can make a transcription product produce multiple proteins, that is, two proteins, the upstream product and the downstream product, are formed by self-cleavage.

The nucleotide sequence of the IFNα gene can be 1552-2118 of SEQ ID No.3, and the amino acid sequence of the IFNα (α interferon) encoded by it can be SEQ ID No.5.

The nucleotide sequence of the promoter element gene can be 2119-2661 of SEQ ID No.3, wherein 2136-2635 of SEQ ID No.3 is the promoter.

The encoding genes of the chimeric switch receptor can be the encoding genes of the TRII extracellular region (TGFβ receptor type II extracellular region), Linker (linker), μPD-1, CD27 transmembrane region and cytoplasmic region from N-terminus to C-terminus. Furthermore, the encoding genes of the transmembrane region and cytoplasmic region of the CD27 can be oCD27 obtained by codon optimization, which is more suitable for human cell expression when the coding amino acid sequence remains unchanged. The nucleotide sequence of the encoding genes for the chimeric switch receptor may be 2662-3882 of SEQ ID No.3 (i.e., the nucleotide sequence shown in SEQ ID No.2), and the amino acid sequence encoding the chimeric switch receptor (CSR) may be SEQ ID No.1.

In the encoding genes of the chimeric switch receptor , the nucleotide sequence of the TRII extracellular region (TGFβ receptor type II extracellular region) encoding gene may be 2662-3159 of SEQ ID No.3, the nucleotide sequence of the Linker encoding gene may be 3160-3219 of SEQ ID No.3, the nucleotide sequence of the μPD-1 encoding gene may be 3220-3657 of SEQ ID No.3, and the nucleotide sequence of the CD27 transmembrane region and cytoplasmic region (i.e., oCD27) encoding gene may be 3658-3882 of SEQ ID No.3.

The chimeric antigen receptor described herein can specifically kill GPC3+ tumor cells. GPC3 is the full name of Glypican-3, which is a member of the heparan sulfate proteoglycan family. It is connected to the cell surface through the glycosylphosphatidylinositol anchor on the cell membrane. It is highly expressed in most liver cancer tissues and can be used to target liver cancer cells to initiate tumor killing. It is an ideal target for tumor treatment.

The IFNα (α interferon) described in this article can have a broad spectrum of anti-tumor effects, directly regulate the proliferation, apoptosis and migration of tumor cells; inhibit the angiogenesis and metastasis of tumors; have immunomodulatory functions, and can enhance anti-tumor immune activity in the following ways: upregulating the expression of MHC I, tumor antigens and PD-L1 in tumor cells; activating natural immune cells such as NK, DC and γδT cells, especially the expansion, migration and function of NK cells; regulating adaptive immune cells such as T cells and B cells, especially the expansion, migration, function and survival of T cells; negatively regulating immunosuppressive cells such as TAM, Treg and myeloid-derived suppressor cells MDSCs; upregulating the expression of PD-L1 in tumor cells.

The chimeric switch receptor described in this article can inhibit the PD-1 and TGFβ signaling pathways to enhance the CD27 signaling pathway, endowing CAR-T cells with the ability to resist the immunosuppressive tumor microenvironment and making them more persistent in vivo; since tumor cells usually express PD-L1 on their surface to activate the inhibitory PD-1 on T cells to circumvent/inhibit anti-tumor T cell responses, the chimeric switch receptor converts the above inhibitory effect into a CD27-mediated activation signal, and at the same time, PD-1 targeting is conducive to the aggregation of CAR-T cells to tumor sites expressing PD-L1.

Although the present invention constructs a GPC3 CAR-IC gene with a nucleotide sequence of SEQ ID No.3, the present invention is not limited to this specific sequence. Those skilled in the art can replace the P2A gene and/or the promoter element gene in the GPC3 CAR-IC gene, for example, using F2A (VKQTLNFDLLKLAGCVESNPG, SEQ ID No.6), T2A (EGRGSLLTCGDVEENPG, SEQ ID No.7), E2A (QCTNYALLKLAGDVESNPG, SEQ ID No.8) and the like which have the same "self-cleavage" function as the P2A gene, but not limited thereto. The promoter element gene can use a promoter known in the art. As long as the constructed nucleic acid molecule can express a chimeric antigen receptor with an amino acid sequence of SEQ ID No.4, an IFNα with an amino acid sequence of SEQ ID No.5, and a fusion protein (chimeric switch receptor) with an amino acid sequence of SEQ ID No.1, and has the same function as the nucleic acid molecule described in the present invention, they are all regarded as nucleic acid molecules equivalent to the nucleic acid molecule of the present invention, and these equivalent nucleic acid molecules do not depart from the protection scope of the present invention.

After extensive and in-depth research, the inventors of this application designed a fusion gene containing an encoding gene of a chimeric antigen receptor and an encoding gene of a chimeric switch receptor , and designed and constructed a GPC3 CAR-IC gene (SEQ ID No. 3) targeting GPC3, which was packaged and infected by retrovirus to obtain GPC3 CAR-IC T cells. The GPC3 CAR-IC T cells constructed by the present invention express two artificial receptors, one of which is a chimeric antigen receptor (CAR), which is used to recognize tumor-associated antigens, specifically kill GPC3+ tumor cells, and target liver cancer cells to initiate tumor killing. The other is a chimeric switch receptor (CSR), which is used to convert immunosuppressive signals in the tumor microenvironment into activation signals in CAR-T cells. The TRII extracellular region can bind to TGFβ, which is secreted in large quantities in HCC, block its inhibitory pathway, and activate the CD27 signaling pathway in CAR-T cells, providing growth stimulation for CAR-T cells, enhancing the survival and migration of CAR-T cells, and inhibiting the tumor microenvironment; μPD-1 in CSR can also bind to PD-L1 expressed in HCC, block the PD-1 signaling pathway on T cells, inhibit T cell exhaustion, and activate the CD27 signaling pathway in CAR-T cells to enhance the tumor killing effect. In addition, the GPC3 CAR-IC T cells constructed by the present invention also express IFNα, which is used for broad-spectrum anti-tumor, reducing antigen heterogeneity and tumor cell escape, enhancing immune cell infiltration, immunomodulation, improving tumor microenvironment, and resisting HBV/HCV infection.

The experimental results show that the GPC3 CAR-IC T cells constructed by the present invention have higher IFN-γ secretion levels, can secrete high levels of IFN-α2 under antigen stimulation, have stronger specific cytotoxicity, and have stronger killing effects on GPC3+ target cells compared with control cells that do not express IC genes (IFNα gene + promoter element gene + chimeric switch receptor encoding gene), and the IFN-α2 secreted by them can induce NK cells to secrete functional effector molecules IFN-γ. The results of the in vivo tumor killing experiment of the HepG2 subcutaneous tumor-bearing NSG mouse model showed that after administration (GPC3-IC CAR-T cells), the secretion of IFN-α2 and IFN-γ in the mouse serum increased significantly, indicating that the GPC3 CAR-IC T cells of the present invention have faster and better tumor suppression effects and stronger in vivo tumor killing activity.

The present invention constructs CAR-T cells by designing a fusion gene containing an encoding gene of a chimeric antigen receptor and an encoding gene of a chimeric switch receptor, converting the inhibitory anti-tumor T cell response activated by PD-L1 and TGFβ into a CD27-mediated activation signal, giving CAR-T cells the ability to resist the immunosuppressive tumor microenvironment, and making them more persistent in vivo, promoting the expansion of CAR-T cells, prolonging the survival time in vivo, and promoting cytokine secretion, and achieving the regulation of the microenvironment near the tumor by promoting the secretion of cytokines IFN-γ and IFN-α2, relieving its immunosuppression, and participating in the killing of tumor cells by mobilizing the body's own immunity. The anti-tumor effect of CAR-T cells is improved, which has important significance and wide application value for CAR-T treatment of tumors (such as hepatocellular carcinoma).

## BRIEF DESCRIPTION OF THE DRAWINGS

Figure 1 is a schematic diagram of the GPC3 CAR-IC gene structure.

Figure 2 is the CAR expression detection result in example 2.

Figure 3 is the cell function detection result of CAR-IC cells secreting IFN-γ (A in figure 3) and IFN-α2 (B in figure 3) in example 3.

Figure 4 is the cytotoxic (degranulated CD107a) function detection result of CAR-IC cells in example 4.

Figure 5 is the CAR-T cell cytotoxicity detection result in example 5. A in Figure 5 is the killing effect on the target cell HepG2, and B in figure 5 is the killing effect on the negative control cell U87 MG.

Figure 6 is the in vitro detection of the induction effect of IFN-α2 secreted by GPC3 CAR-IC T cells co-cultured with target cells on NK cells in example 6.

Figure 7 is the evaluation of the in vivo tumor killing effect and animal survival of the transplanted tumor HepG2-NSG model in example 7.

## DETAILED DESCRIPTION

The present invention is further described in detail below in conjunction with specific embodiments. The examples given are only for illustrating the present invention, not for limiting the scope of the present invention. The examples provided below can serve as a guide for further improvements by ordinary technicians in the field of this technology, and do not constitute a limitation of the present invention in any way.

The experimental methods in the following examples, unless otherwise specified, are all conventional methods, and are carried out in accordance with the techniques or conditions described in the literature in the field or in accordance with the product instructions. The materials, reagents, etc. used in the following examples, unless otherwise specified, can all be obtained from commercial channels.

The pUC57 vector in the following examples is a product of Beijing Tsingke Biotech Co., Ltd.

The retroviral vector MP71 in the following examples is recorded in the following document: Engels B, Cam H, et al. Retroviral Vectors for High-Level Transgene Expression in T Lymphocytes [J]. Human Gene Therapy, 2003, 14(12):1155-1168. The public can obtain the biological material from the applicant. The biological material is only used to repeat the experiment of the present invention and cannot be used for other purposes.

The human peripheral blood mononuclear cells (PBMC) in the following examples are derived from the venous blood of healthy volunteers.

The HepG2 cells in the following examples are products of the cell bank of the Chinese Academy of Sciences, with the catalog number SCSP-510.

The U87 MG cells in the following examples are products of the cell bank of the Chinese Academy of Sciences, with the catalog number TCHu138.

The NSG mice (6-7 weeks old, weighing 18-25 g) in the following examples were purchased from Biocytogen Jiangsu Gene Biotechnology Co., Ltd.

The RPMI-1640 culture medium in the following examples is a product of Sigma, with the catalog number R8758.

The RPMI-1640 culture medium containing 10% fetal bovine serum (FBS) (10% FBS RPMI-1640 culture medium) in the following examples is prepared with RPMI-1640 culture medium (R8758) as solvent and fetal bovine serum as solute, also known as complete medium.

**Example 1: CAR-IC cell construction**

The CAR-IC cells constructed in this embodiment target GPC3 and express two artificial receptors, one is a chimeric antigen receptor (CAR) for the recognition of tumor-associated antigens, and the other is a chimeric switch receptor (CSR) for converting immunosuppressive signals in the tumor microenvironment into activation signals in CAR-T cells, thereby providing growth stimulation for CAR-T cells, enhancing the survival and migration of CAR-T cells, and inhibiting the tumor microenvironment. The specific construction method is as follows:

1. GPC3 CAR-IC gene design

The GPC3 CAR-IC gene is composed of the encoding gene of the chimeric antigen receptor, the P2A gene, the IFN$\alpha$ gene, the promoter element gene, and the encoding gene of the chimeric switch receptor from the N-terminus to the C-terminus. The structural schematic diagram is shown in Figure 1 (the three parts of the IFN$\alpha$ gene, the promoter element gene, and the encoding gene of the chimeric switch receptor are collectively referred to as the IC gene). Wherein: The nucleotide sequence of the IC gene (IFN$\alpha$ gene + promoter element gene+ chimeric switch receptor encoding gene) is 1552-3882 of SEQ ID No.3.

The encoding genes of the chimeric antigen receptor are Signal (signal peptide, GPC3 scFv (tumor antigen binding region, i.e. single-chain antibody region), Hinge (hinge region), CD8 (transmembrane region), 4-1BB (intracellular signal region), and CD3$\zeta$ (intracellular signal region) from N-terminus to C-terminus. The nucleotide sequence of the chimeric antigen receptor encoding gene is 1-1473 of SEQ ID No.3, and the amino acid sequence encoding the chimeric antigen receptor (CAR) is SEQ ID No.4. Among the encoding genes of the chimeric antigen receptor, the nucleotide sequence of encoding gene of Signal (signal peptide is 1-63 of SEQ ID No.3, the nucleotide sequence of encoding gene of GPC3 scFv is 64-789 of SEQ ID No.3, the nucleotide sequence of the encoding gene of Hinge (hinge region) plus CD8 (transmembrane region) is 790-996 of SEQ ID No.3, the nucleotide sequence of encoding gene of 4-1BB (intracellular signal region) is 997-1137 of SEQ ID No.3, and the nucleotide sequence of encoding gene of CD3$\zeta$ (intracellular signal region) is 1138-1473 of SEQ ID No.3.

The nucleotide sequence of the P2A gene is 1474-1551 of SEQ ID No.3. The P2A peptide it encodes is a short peptide derived from a virus, commonly known as a "self-cleaving" peptide. The "self-cleavage" function of the P2A peptide can make a transcription product produce multiple proteins, that is, two proteins, upstream product and downstream product, are formed by self-cleavage.

The nucleotide sequence of the IFN$\alpha$ gene is 1552-2118 of SEQ ID No.3, and the amino acid sequence of IFN$\alpha$ ($\alpha$ interferon) encoded by it is SEQ ID No.5.

The nucleotide sequence of the promoter element gene is 2119-2661 of SEQ ID No.3, among which 2136-2635 of SEQ ID No.3 is the promoter.

The encoding genes of the chimeric switch receptor are the encoding genes of TRII extracellular region (TGF$\beta$ receptor type II extracellular region), Linker, $\mu$PD-1, CD27 transmembrane region and cytoplasmic region from N-terminus to C-terminus. Furthermore, the encoding genes of the CD27 transmembrane region and cytoplasmic region are oCD27 obtained by codon optimization, which is more suitable for human cell expression when the encoded amino acid sequence remains unchanged. $\mu$PD-1 is the mutation-optimized extracellular region of PD-1. The nucleotide sequence of the encoding gene for the chimeric switch receptor is 2662-3882 of SEQ ID No.3 (i.e., the nucleotide sequence shown in SEQ ID No.2), and the amino acid sequence of the chimeric switch receptor (CSR) is SEQ ID No.1. In the encoding gene of the chimeric switch receptor, the nucleotide sequence of the encoding gene of TRII extracellular region (TGF$\beta$ receptor type II extracellular region) is 2662-3159 of SEQ ID No.3, the nucleotide sequence of the encoding gene of Linker is 3160-3219 of SEQ ID No.3, the nucleotide sequence of the encoding gene of $\mu$PD-1 is 3220-3657 of SEQ ID No.3, and the nucleotide sequence of the encoding gene of CD27 transmembrane region and cytoplasmic region (i.e., oCD27) is 3658-3882 of SEQ ID No.3.

The chimeric antigen receptor can specifically kill GPC3[+] tumor cells. GPC3 is the full name of Glypican-3, a member of the heparan sulfate proteoglycan family. It is connected to the cell surface through the glycosylphosphatidylinositol anchor on the cell membrane. It is highly expressed in most liver cancer tissues and can be used to target liver cancer cells to initiate tumor killing. It is an ideal target for tumor treatment.

IFNα (α interferon) can be broad-spectrum anti-tumor, directly regulate the proliferation, apoptosis and migration of tumor cells; inhibit the formation and metastasis of tumor blood vessels; has immunomodulatory function, and can enhance anti-tumor immune activity in the following ways: upregulating the expression of MHC I, tumor antigens and PD-L1 in tumor cells; activating natural immune cells such as NK, DC and γδT cells, especially the expansion, migration and function of NK cells; regulating adaptive immune cells such as T cells and B cells, especially the expansion, migration, function and survival of T cells; negatively regulating immunosuppressive cells such as TAM, Treg and myeloid-derived suppressor cells MDSCs; upregulating the expression of PD-L1 in tumor cells.

The chimeric switch receptor is composed of a TRII-type extracellular region, μPD-1 and CD27 transmembrane and cytoplasmic regions, which can inhibit the PD-1 and TGFβ signaling pathways to enhance the CD27 signaling pathway, endow CAR-T cells with the ability to resist the immunosuppressive tumor microenvironment, and make them more persistent in vivo; since tumor cells usually express PD-L1 on their surface to activate the inhibitory PD-1 on T cells to circumvent/inhibit anti-tumor T cell responses, the chimeric switch receptor converts the above inhibitory effect into a CD27-mediated activation signal, and at the same time, PD-1 targeting is conducive to the aggregation of CAR-T cells to tumor sites expressing PD-L1.

2. GPC3 CAR-IC gene synthesis

The full-length sequence of the wild-type human CD27 transmembrane and cytoplasmic region encoding gene is called nCD27, and oCD27 is obtained by codon optimization to ensure that it is more suitable for human cell expression without changing the encoding amino acid sequence. The nucleotide sequence of encoding gene oCD27 is shown in positions 3658-3882 of SEQ ID No.3.

According to the design in step 1 (see Figure 1), the GPC3 CAR-IC gene was constructed and synthesized by Beijing Tsingke Biotech Co., Ltd., cloned on the pUC57 vector, and the recombinant vector pUC57-GPC3 CAR-IC was obtained and sequenced.

The nucleotide sequence of the GPC3 CAR-IC gene is shown in SEQ ID No. 3, and it expresses a chimeric antigen receptor with an amino acid sequence of SEQ ID No. 4, an IFNα with an amino acid sequence of SEQ ID No. 5, and a fusion protein (chimeric switch receptor) with an amino acid sequence of SEQ ID No. 1.

3. CAR-IC cell construction

The GPC3 CAR-IC gene constructed in step 2 was transferred into T cells for stable expression, and T cells expressing the GPC3 CAR-IC gene (SEQ ID No. 3) were obtained, which were named GPC3 CAR-IC T cells. The specific steps are as follows:

3-1. Construction of recombinant retroviral vector

(1) Double-digest the recombinant vector pUC57-GPC3 CAR-IC with NotI (NEB) and EcoRI (NEB), and recover the target gene fragment by gel cutting.
(2) Double-digest the retroviral vector MP71 with NotI and EcoRI, and recover the large vector fragment by gel cutting.
(3) Use T4 ligase (NEB) to connect the above target gene fragment and the large vector fragment to obtain the recombinant retroviral vector MP71-GPC3 CAR-IC carrying the GPC3 CAR-IC gene.
(4) Transform the recombinant retroviral vector MP71-GPC3 CAR-IC into competent Escherichia coli DH5α, and use Qiagen's plasmid purification kit to extract and purify the plasmid to obtain the MP71-GPC3 CAR-IC plasmid.
(5) According to the above method, simultaneously construct the MP71-GPC3 CAR plasmid without IC gene as a control.

The recombinant retroviral vector MP71-GPC3 CAR-IC (i.e., MP71-GPC3 CAR-IC plasmid) is a recombinant expression vector obtained by replacing the fragment (small fragment) between the NotI and EcoRI recognition sites of the retroviral vector MP71 with a DNA fragment whose nucleotide sequence is SEQ ID No.3 in the sequence list, while keeping the other nucleotide sequences of the retroviral vector MP71 unchanged.

The recombinant retroviral vector MP71-GPC3 CAR (i.e., MP71-GPC3 CAR plasmid) is a recombinant expression vector obtained by replacing the fragment (small fragment) between the NotI and EcoRI recognition sites of the retroviral vector MP71 with a DNA fragment (encoding gene of chimeric antigen receptor) whose nucleotide sequence is shown in 1-1473 of SEQ ID No.3 in the sequence list, while keeping the other nucleotide sequences of the retroviral vector MP71 unchanged.

3-2. Retroviral packaging

The MP71-GPC3 CAR-IC and MP71-GPC3 CAR plasmids prepared in step 3-1 were introduced into packaging cells for packaging, and the virus assembly was completed to obtain retrovirus. The specific steps of virus packaging are as follows:

a) Day 1: Phoenix Ecotropic (ECO) cells should be less than 20 generations and not overgrown. Plate at a cell density of $0.6\times10^6$/ml, add 10 mL of DMEM medium to a 10 cm dish, mix the cells thoroughly, and culture at 37°C overnight;

b) Day 2: ECO cells are transfected when the confluence reaches about 90% (usually about 14-18 hours after plating); prepare 12.5 $\mu$g of plasmid, 250 $\mu$L of 1.25 M $CaCl_2$, and 1 mL of $H_2O$, with a total volume of 1.25 mL; add 2$\times$HBS equal to the volume of the plasmid complex to another tube, and vortex for 20 s while adding the plasmid complex. Gently add the mixture along the edge to the ECO dish, incubate at 37°C for 4 h, remove the culture medium, wash once with PBS, and re-add preheated fresh culture medium;

c) Day 4: collect the supernatant 48 h after transfection, filter with a 0.45 $\mu$m filter to obtain the retroviral solution, and store it at -80°C;

d) Add 1.2 mL of 15 ug/mL Retronectin coating solution to each well of the NTC 6-well plate and incubate at 4°C overnight;

e) carefully remove the blocking solution, add 2 mL/well PBS to wash, add 5 mL of the above virus solution to each well, centrifuge at 32°C and 2000$\times$g for 2 h, and discard the unbound virus supernatant;

f) take the logarithmic phase PG13 cells, rinse once with 10 mL PBS, add 1 mL 0.25% recombinant trypsin, and let it stand at room temperature for 2-3 min;

g) add 5 ml of complete culture medium containing 10% FBS to terminate digestion, centrifuge at 1500 rpm for 5 min;

h) discard the supernatant, adjust the cell density to $0.5\times10^5$ cell/mL with complete culture medium, and add 3 mL/well to the above virus-coated NTC 6-well plate to make the final cell number $1.5\times10^5$ cell/well;

i) centrifuge at 1000 rpm for 1 min, and culture at 37°C, 5% CO2 for 48 h;

j) after 1~2 generations of passage, transfer to T175 culture flask and culture with DEME medium containing 12% FBS for 2 d;

k) replace new DEME medium containing 12% FBS and continue to culture for 48 h, then collect the supernatant, filter with 0.45 $\mu$m filter to obtain retrovirus solution, and store it in aliquots at -80°C.

3-3. Retrovirus-infected human T cells

a) thaw frozen healthy human peripheral blood PBMC, adjust the cell density to $1\times10^6$-$2\times10^6$ cells/mL with RPMI-1640 medium containing 10% fetal bovine serum (FBS).

b) PBMCs were collected with Ficoll separation solution (Tianjin Haoyang), CD3[+] T cells were separated by magnetic beads, and clinical-grade Dynabeads Human T Expander CD3/CD28 magnetic beads (Invitrogen) were added to activate T cells at a ratio of 3:1.

c) On the second day after T cell activation, RetroNectin (TAKARA) diluted to a final concentration of 15 $\mu$g/mL with PBS was coated on non-tissue-treated culture plates, 1.2 mL per well of a 6-well plate. Protect from light and store overnight at 4°C.

d) After two days of T cell activation culture, the coated 6-well plate was removed, the coating solution was aspirated, and PBS was added to wash the plate once.

e) The retrovirus solution prepared in step 3-2 was added to the wells, 5-6 mL per well, and centrifuged at 32°C and 2000$\times$g for 2 h. 3 mL of fresh complete medium containing hIL-2 (500 U/mL) was added to each well and cultured for 1 day.

f) After cell infection, observe the cell density every day, and add T cell culture medium containing 100 U/mL IL-2 in time to maintain the density of T cells at about $5\times10^5$/mL to facilitate cell expansion.

g) CAR-IC cells and control CAR T cells infected with the retrovirus prepared in step 3-2 are obtained, and are named GPC3 CAR-IC T cells (i.e., T cells expressing the GPC3 CAR-IC gene with a nucleotide sequence of SEQ ID No.3) and GPC3 CAR T cells as a control (i.e., T cells expressing the DNA molecule shown in positions 1-1473 of SEQ ID No.1 with a nucleotide sequence of SEQ ID No.1).

**Example 2. CAR expression detection of CAR-IC cells**

The GPC3 CAR-IC T cells and GPC3 CAR T cells prepared in example 1, as well as CTR T cells (i.e., T cells without virus transfection, as a control) were cultured at 37°C in RPMI-1640 medium containing 10% fetal bovine serum (FBS), and recorded as D0 day. CAR expression was detected until D8 day.
1. GPC3 CAR expression detection:
(1) After centrifugation (1500 rpm×5 min), discard the supernatant, add 200 μL FACS buffer (1×PBS containing 0.1% NaN$_3$ and 2% FBS) to each well of the 96-well round bottom plate, resuspend, and centrifuge at 1500 rpm for 5 min;
(2) Add 60 μL of the premix of prepared fluorescent-labeled antibody and fluorescent-labeled recombinant protein to each well (see Table 1 for the preparation of the premix, wherein the fluorescent-labeled antibody is the fluorescent-labeled anti-human CD3/CD4/CD8 antibody, and the fluorescent-labeled recombinant protein (rp) is the FITC-labeled GPC3 recombinant protein), resuspend and mix, and incubate at 4°C for 30 min;

Table 1. Preparation of premix

| Label | Fluorescent Label | Catalog No. | Brand | Dilution | Volume (μL) |
|---|---|---|---|---|---|
| CD3 | PE | 344806 | Biolegend | 1:100 | 0.6 |
| CD4 | APCCy7 | 317450 | Biolegend | 1:200 | 0.3 |
| CD8a | PECy7 | 301012 | Biolegend | 1:200 | 0.3 |
| GPC3 recombinant protein | FITC | GP3-HF258 | Acrobiosystems | 1:50 | 1.2 |

(3) Add 200 μL FACS buffer to each well and centrifuge at 1500 rpm for 5 min;
(4) Discard the supernatant, resuspend the cells in 400 μL FACS buffer and transfer them to a flow cytometer (BDCanto-II) to read the cells and analyze the percentage of GPC3 CAR in T cells.
2. Detection of interferon α2 (IFNα2) expression:

(1) after centrifugation (1500 rpm×5 min), discard the supernatant, add 200 μL FACS buffer (1×PBS containing 0.1% NaN$_3$ and 2% FBS) to each well of the 96-well round bottom plate, resuspend, and centrifuge at 1500 rpm for 5 min;
(2) add 60 μL of the premix of prepared fluorescent-labeled antibody and fluorescent-labeled recombinant protein to each well (see Table 1 for the preparation of the premix, where the fluorescent-labeled antibody is the fluorescent-labeled anti-human CD3/CD4/CD8 antibody, and the fluorescent-labeled recombinant protein (rp) is the FITC-labeled GPC3 recombinant protein) and resuspend and mix, and incubate at 4°C for 30 min;
(3) add 200 μL FACS buffer to each well, centrifuge at 1500 rpm for 5 min;
(4) discard the supernatant, add 150 μL to each well Cytofix/Cytoperm (BD, Catalog No. 55472) was resuspended and mixed, and incubated at room temperature for 15 min in the dark;
(5) After centrifugation at 1500 rpm for 5 min, the supernatant was discarded, and 200 μL Perm/Wash buffer (BD, Catalog No. 554723) was added to each well and resuspended and mixed, and centrifuged at 1500 rpm for 5 min, and washed twice by centrifugation;
(6) 20 μL of prepared anti-human IFN-α2 was added to each well and resuspended and mixed, and incubated at room temperature in the dark for 20 min;
(7) 200 μL of 0.5% BSA was added to each well and centrifuged at 1500 rpm for 5 min, and washed twice by centrifugation;
(8) 20 μL of prepared SA-APC was added to each well and resuspended and mixed, and incubated at 4°C for 10 min;
(9) 200 μL of 0.5% BSA was added to each well and centrifuged at 1500 rpm for 5 min. After discarding the supernatant, resuspend the cells in 400 μL FACS buffer and transfer them to a flow cytometer. Read the cells using a flow cytometer (BDCanto-II) to analyze the percentage of IFNα2 in T cells.

3. μPD-1/TRII expression detection:

(1) after centrifugation (1500 rpm×5 min), discard the supernatant, add 200 μL FACS buffer (1×PBS containing 0.1% NaN$_3$ and 2% FBS) to each well of the 96-well round bottom plate, resuspend, and centrifuge at 1500 rpm for 5 min;
(2) add 60 μL of the premix of prepared fluorescent-labeled antibody and fluorescent-labeled recombinant protein to each well (see Table 1 for the preparation of the premix, where the fluorescent-labeled antibody is the fluorescent-labeled anti-human CD3/CD4/CD8 antibody, and the fluorescent-labeled recombinant protein (rp) is the FITC-labeled GPC3 recombinant protein) to each well, resuspend and mix, and incubate at 4°C for 30 min;
(3) add 200 μL FACS buffer to each well, centrifuge at 1500 rpm for 5 min;

(4) discard the supernatant and use 400 μL FACS The cells were resuspended in buffer and transferred to a flow cytometer. The cells were read by flow cytometer (BDCanto-II) to analyze the percentage of μPD-1/TRII in T cells.

The test results are shown in Figure 2. CAR (i.e., GPC3 CAR), IFNα2, μPD-1 and TRII were simultaneously expressed in CAR-IC cells (i.e., GPC3 CAR-IC T cells prepared in Example 1), indicating that the expression of CAR molecules in CAR-IC cells reached the expected design.

**Example 3. Cell function detection of IFN-γ and IFN-α2 secretion by CAR-IC cells**

The test cells are GPC3 CAR-IC T cells and GPC3 CAR T cells prepared in example 1, as well as CTR T cells (i.e., T cells without virus transfection).

The above test cells were cultured until D10, and $1 \times 10^6$ cells were added to a 24-well TC plate at 1 mL per well. HepG2 cells were added at a ratio of test cells: target cells = 1:1, and co-cultured in a 37°C incubator, and recorded as D0.

The detection groups and controls are as follows:

CTR T+Medium: 1 mL of Medium (10% FBS RPMI-1640 medium) was added to 1 mL of non-virus-transfected T cells (CTR T cells) per well, with a total of $1 \times 10^6$ cells;

CTR T+HepG2: 1 mL of HepG2 cells was added to 1 mL of non-virus-transfected T cells (CTR T cells) per well, with a total of $1 \times 10^6$ cells;

GPC3 CAR T+Medium: 1 mL of Medium (10% FBS RPMI-1640 medium) was added to 1 mL of conventional GPC3-targeted CAR T cells (GPC3 CAR T cells) per well;

GPC3 CAR T+HepG2: 1 mL of HepG2 cells was added to 1 mL of conventional GPC3-targeted CAR T cells (GPC3 CAR T cells) per well, with a total of $1 \times 10^6$ cells;

GPC3 CAR-IC T+Medium: 1 mL of Medium (10% FBS RPMI-1640 medium) was added to $1 \times 10^6$ CAR-IC T cells targeting GPC3 (GPC3 CAR-IC T cells) per well;

GPC3 CAR-IC T+HepG2: 1 mL of $1 \times 10^6$ HepG2 cells were added to 1 mL of CAR-IC T cells targeting GPC3 (GPC3 CAR-IC T cells) per well.

The above co-cultured cells were taken for three consecutive days (D1, D2, and D3) to detect the secreted IFN-γ and IFN-α2.

The specific detection steps are as follows:

1. Adjust the cell density of the co-cultured cells cultured on D1, D2, and D3 to $2 \times 10^6$ cells/mL, take 100 μL and add it to a 96-well U-bottom plate, add Brefeldin A (Med Chem Express, HY-16592) with a final concentration of 5 μg/mL to each well, and incubate in a 37°C incubator for 5 to 6 hours.

2. After incubation, flow cytometry staining was performed. The operation steps are as follows:

(1) after centrifugation (1500 rpm×5 min), the cells were discarded and the supernatant was added to each well (1×PBS containing 0.1% NaN₃ and 2% FBS), resuspended, and centrifuged at 1500 rpm for 5 min. This step was repeated twice;

(2) 60 μL of the premix of prepared fluorescent-labeled antibody and fluorescent-labeled recombinant protein was added to each well (the preparation of the premix is shown in Table 1, where the fluorescent-labeled antibody is the fluorescent-labeled anti-human CD3/CD4/CD8 antibody, and the fluorescent-labeled recombinant protein (rp) is the FITC-labeled GPC3 recombinant protein), resuspended and mixed, and incubated at room temperature for 10 min in the dark;

(3) 200 μL of FACS buffer was added to each well, centrifuged at 1500 rpm for 5 min, and the supernatant was discarded;

(4) 150 μL of Cytofix/Cytoperm (BD, Catalog No. 55472) was resuspended and mixed, and incubated at room temperature for 15 min in the dark;

(5) After centrifugation at 1500 rpm for 5 min, the supernatant was discarded, and 200 μL Perm/Wash buffer (BD, Catalog No. 554723) was added to each well, and the cells were resuspended and mixed. Centrifugation was performed at 1500 rpm for 5 min, and the cells were washed twice by centrifugation;

(6) 20 μL diluted APC-labeled anti-human IFN-γ (Biolegend, Catalog No. 506510) or anti-human IFN-α2 (Biolegend, Catalog No. 537204) was added to each well, and the cells were resuspended and mixed. The cells were incubated at room temperature for 20 min in the dark;

(7) 200 μL Perm buffer was added to each well, and the cells were centrifuged at 1500 rpm for 5 min. After discarding the supernatant, resuspend the cells with 400 μL FACS buffer and transfer them to a flow cytometer. Read the cells with a flow cytometer (BD Canto-II) to analyze the percentage of functional effector molecules IFN-

13

γ and IFN-α2 in GPC3 CAR-IC T cells and control cells.

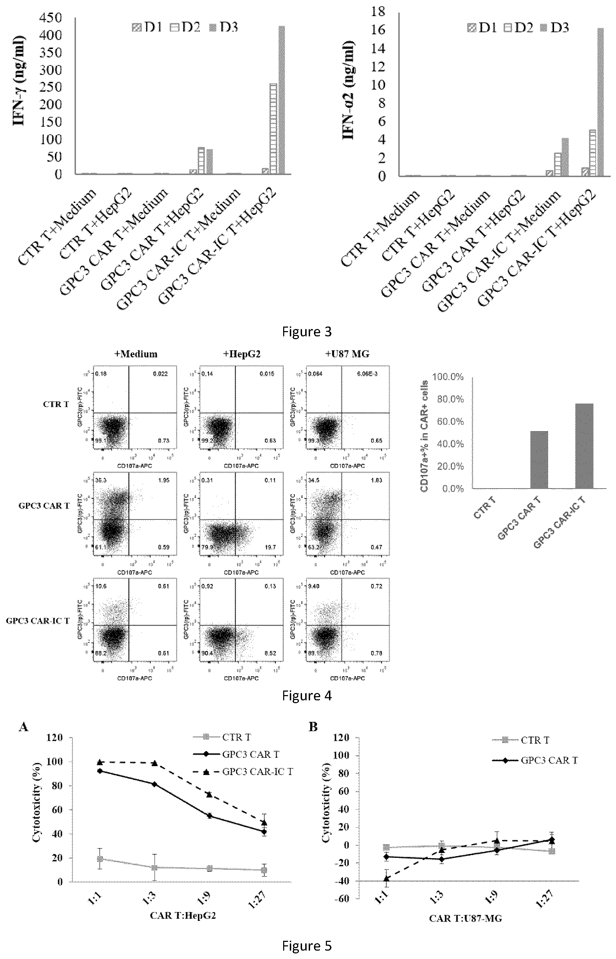

The detection results are shown in Figure 3. After co-culture with target cells (HepG2 cells), both GPC3 CAR T cells and GPC3 CAR-IC T cells can specifically secrete T cell effector molecule IFN-γ; compared with GPC3 CAR T cells, GPC3 CAR-IC T cells have a higher level of IFN-γ secretion. In addition, GPC3 CAR-IC T cells can secrete low levels of IFN-α2 without antigen stimulation; GPC3 CAR-IC T cells can secrete high levels of IFN-α2 under antigen stimulation.

**Example 4. Cytotoxicity (degranulation CD107a) function detection of CAR-IC cells**

The degranulation ability of CAR-IC cells, i.e., the expression of CD107a, was detected. The test cells were GPC3 CAR-IC T cells and GPC3 CAR T cells prepared in Example 1, as well as CTR T cells (i.e., T cells without virus transfection).

1) The above test cells were cultured until D10, the cell density was adjusted to $2 \times 10^6$ cells/mL, and 100 μL was added to a 96-well U-bottom plate;
2) 100 μL of $2 \times 10^5$ HepG2 cells were added to each well at a ratio of test cells: target cells = 1:1 as CAR antigen-specific stimulation (+HepG2) or negative control U87 MG cells (+U87 MG), and 100 μL Medium (10% FBS RPMI-1640 medium) was added to each well as a negative control (+Medium);
3) 1 μL of APC-labeled anti-human CD107a antibody (Biolegend, Cat. No. 328620) was added to each well and incubated at 37°C for 1 h;
4) 10 μL of 1:50 diluted Monensin Solution (Invitrogen, Cat. No. 00-4505-51) was added to each well and incubated for 3 h;
5) After incubation, flow cytometry staining was performed, and the operation steps were as follows:

(1) after centrifugation of the cells (1500 rpm×5 min), the supernatant was discarded, 200 μL FACS buffer (1×PBS containing 0.1% NaN$_3$ and 2% FBS) was added to each well, resuspended and mixed, centrifuged at 1500 rpm for 5 min, and this step was repeated twice;
(2) 60 μL of the premix of prepared fluorescent-labeled antibody and fluorescent-labeled recombinant protein (premix preparation see Table 1, where the fluorescent-labeled antibody is the fluorescent-labeled anti-human CD3/CD4/CD8 antibody, and the fluorescent-labeled recombinant protein (rp) is the FITC-labeled GPC3 recombinant protein) was added to each well, resuspended and mixed, and incubated at room temperature for 10 min away from light;
(3) Centrifuged at 1500 rpm for 5 min. After discarding the supernatant, resuspend the cells with 400 μL FACS buffer and transfer them to a flow cytometer. Read the cells with a flow cytometer (BD Canto-II) and analyze the percentage of cell degranulation molecule CD107a in T cells.

The test results are shown in Figure 4. After co-culture with target cells, both GPC3 CAR T cells and GPC3 CAR-IC T cells can specifically upregulate CD107a degranulation. In addition, compared with GPC3 CAR-T cells, GPC3 CAR-IC T cells have a stronger specific cytotoxic effect (CD107a).

**Example 5. CAR-T cell cytotoxicity assay**

The test cells are GPC3 CAR-IC T cells and GPC3 CAR T cells prepared in Example 1, as well as CTR T cells (i.e., T cells without virus transfection).

1) The above test cells were cultured until D10, the cell density was adjusted to $2 \times 10^5$ cells/mL, 100 μL was taken and added to a 96-well U-bottom plate; D-firefly luciferin sodium salt (Yeasen Biotechnology, catalog number 40901ES08, storage concentration is 100 mg/mL) was added at a final concentration of 100 μg/mL, and repeated for 3 wells;
2) Target cells HepG2 or negative control cells U87 MG (the cell density of target cells under 1:1 condition is $2 \times 10^4$ cells) were added at different effector-target ratios (1:1, 1:3, 1:9, 1:27), and cultured at 37°C for 16 h.
3) Measure the fluorescence value with TECAN spark microplate reader, and take the average value of three replicate wells to calculate the specific cytotoxicity of CAR-T cells:

$$\text{Specific lysis \%} = 100 - 100 \times (\text{Eexp} - \text{Emin})/(\text{Tmax} - \text{Tmin})$$

Eexp: RLU value when effector cells and target cells are co-cultured;
Emin: RLU value of spontaneous death of effector cells in the absence of cells;

Tmax: RLU value of spontaneous death of target cells in the absence of effector cells;

Tmin: RLU value under the condition of maximum killing rate.

The test results are shown in Figure 5. Both GPC3 CAR T cells and GPC3 CAR-IC T cells can specifically kill GPC3+ target cells (HepG2 cells) and have no killing effect on GPC3-target cells (U87 MG cells); the control CTR T cells have no killing effect on GPC3+ target cells and GPC3-target cells. In addition, GPC3 CAR-IC T cells had a stronger killing effect on GPC3+ target cells (HepG2 cells) compared with GPC3 CAR-T cells.

**Example 6. In vitro detection of the induction effect of IFN-α2 secreted by GPC3 CAR-IC T cells co-cultured with target cells on NK cells**

The test cells were GPC3 CAR-IC T cells and GPC3 CAR T cells prepared in example 1, and CTR T cells (ie, T cells without virus transfection). The detection steps are as follows:

1) The above-mentioned test cells were cultured until D10, the cell density was adjusted to $2\times10^6$ cells/mL, and 1 mL was added to a 96-well U-bottom plate;

2) 1 mL of $2\times10^6$ HepG2 cells were added to each well at a ratio of test cells: target cells = 1:1 and co-cultured for 3 days;

3) the supernatant was collected by centrifugation and co-incubated with PBMC cells, where PBMC: $2\times10^5$/well, 0.1 mL/well; supernatant: 0.1 mL/well;

4) after incubation overnight, Brefeldin A (Med Chem Express, HY-16592) was added to each well to a working concentration of 5 μg/mL and incubated at 37°C for 4 h;

5) After incubation, flow cytometry staining was performed, and the operation steps were as follows:

(1) after cell centrifugation (1500 rpm×5 min), the supernatant was discarded and 200 μL FACS buffer (1×PBS containing 0.1% NaN$_3$ and 2% FBS) and centrifuge at 1500 rpm for 5 min;

(2) add 40 μL of prepared fluorescent antibody (fluorescently labeled anti-human CD3/CD56) to each well, resuspend and mix, incubate at room temperature in the dark for 10 min;

(3) add 200 μL of FACS buffer to each well, centrifuge at 1500 rpm for 5 min, and discard the supernatant;

(4) add 150 μL of Cytofix/Cytoperm (BD, Cat. No. 55472) to each well, resuspend and mix, incubate at room temperature in the dark for 15 min;

(5) centrifuge at 1500 rpm for 5 min, discard the supernatant, add 200 μL of Perm/Wash buffer (BD, Cat. No. 554723) to each well, resuspend and mix, centrifuge at 1500 rpm for 5 min, and wash twice;

(6) add 40 μL of prepared fluorescent antibody (fluorescently labeled anti-human IFNy/Granzyme B) Resuspend and mix, incubate at room temperature for 20 min away from light;

(7) add 200 μL Perm buffer to each well and centrifuge at 1500 rpm for 5 min. After discarding the supernatant, resuspend the cells in 400 μL FACS buffer and transfer them to a flow cytometer. Read the cells using a flow cytometer (BDCanto-II) to analyze the percentage of effector molecules IFN-γ and Granzyme B in NK cells.

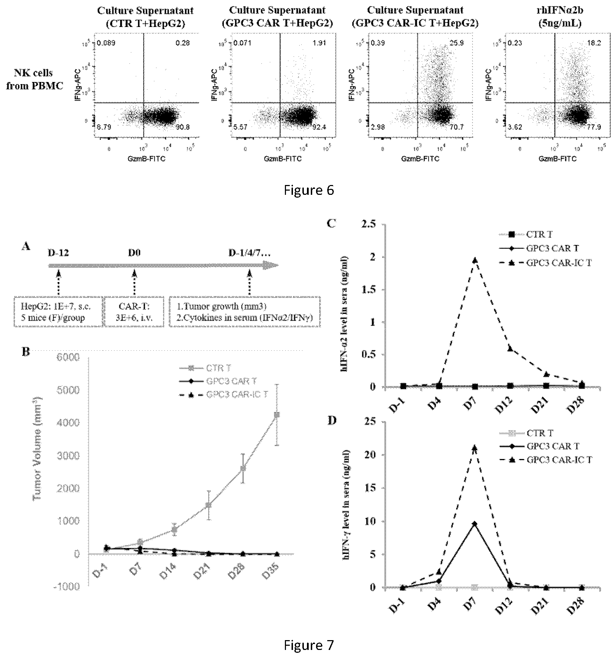

The test results are shown in figure 6. IFN-α2 secreted by GPC3 CAR-IC T cells can induce NK cells to secrete the functional effector molecule IFN-γ.

**Example 7. Evaluation of the in vivo tumoricidal effect and animal survival of GPC3 CAR-IC T cells in the HepG2-NSG transplant model**

The HepG2 subcutaneous tumor-bearing NSG mouse model (HepG2-NSG model) was used to evaluate the in vivo tumoricidal effect of GPC3 CAR-IC T cells and animal survival. The specific steps are as follows:

1) NSG mice (6-7 weeks old) were subcutaneously inoculated with HepG2 cells, $1\times10^7$ cells/mouse, on the back of the right lower limb.

2) On day 12, when the mouse tumor grew to 80-100 mm3, GPC3 CAR-IC T cells were injected, which was recorded as D0. During this period, the tumor-bearing mice were randomly divided into 3 groups, 5 mice/group, and the groups were divided as follows:

Control group (CTR T): injection of CTR T cells, $3\times10^6$ cells/mouse;

GPC3 CAR T group: injection of GPC3 CAR-T cells, $3\times10^6$ cells/mouse;

GPC3 CAR-IC T group: injection of GPC3-IC CAR-T cells, $3\times10^6$ cells/mouse.

3) Tumor size was measured with a vernier caliper on D7, D14, D21, D28, and D35 days after administration.

4) Blood was collected from the submandibular area of the mice 1 day before administration (D-1) and on D4, D7, D12, D21, and D28 days after administration to collect mouse serum, and the IFN-γ and IFN-α2 secreted by GPC3 CAR-IC T cells and control cells in the mice were detected by serum multiple cytokine method.

The test results are shown in Figure 7. Compared with GPC3 CAR-T cells, the tumor inhibition effect of GPC3 CAR-IC T cells on D7/D14 days was faster and better. There was a high level of IFN-α2 secretion in the serum of mice adoptively transferred with GPC3 CAR-IC T cells. Compared with adoptively transferred GPC3 CAR-T mice, there was a higher level of IFN-γ secretion in the serum of GPC3 CAR-IC T mice. This shows that GPC3 CAR-IC T cells have stronger tumoricidal activity in vivo.

The present invention is described in detail above. For those skilled in the art, the present invention can be implemented in a wide range under equivalent parameters, concentrations and conditions without departing from the purpose and scope of the present invention and without unnecessary experiments. Although the present invention provides specific embodiments, it should be understood that the present invention can be further improved. In short, according to the principles of the present invention, the present application intends to include any changes, uses or improvements to the present invention, including changes made by conventional techniques known in the art that are out of the scope disclosed in the present application. Some basic features can be applied within the scope of the following claims.

## Industrial Application

After extensive and in-depth research, the inventors of the present application designed a fusion gene containing an encoding gene of a chimeric antigen receptor and an encoding gene of a chimeric switch receptor, and designed and constructed a GPC3 CAR-IC gene (SEQ ID No. 3) targeting GPC3, which was packaged and infected by retrovirus to obtain GPC3 CAR-IC T cells. The GPC3 CAR-IC T cells constructed in the present invention specifically kill GPC3$^+$ tumor cells, have good tumor killing effect, faster and better tumor inhibition effect, and have stronger in vivo tumor killing activity. They are suitable for broad-spectrum anti-tumor, reducing antigen heterogeneity and tumor cell escape, enhancing immune cell infiltration, immune regulation, improving tumor microenvironment, and resisting HBV/HCV infection. They are of great significance and wide application value for CAR-T treatment of tumors (such as hepatocellular carcinoma).

## Claims

1. A nucleic acid molecule, wherein the nucleic acid molecule comprises an encoding gene of a chimeric antigen receptor and an encoding gene of a chimeric switch receptor.

2. The nucleic acid molecule according to claim 1, wherein the chimeric switch receptor comprises a TGFβ receptor type II extracellular region, μPD-1 and a CD27 transmembrane region and a cytoplasmic region, and the amino acid sequence of the μPD-1 is positions 187-332 of SEQ ID No. 1.

3. The nucleic acid molecule according to claim 2, wherein the encoding gene of the chimeric switch receptor is any one of the following:

   B1) a nucleic acid molecule encoding a fusion protein;
   B2) a DNA molecule whose encoding sequence is SEQ ID No.2;
   B3) a DNA molecule whose nucleotide sequence is SEQ ID No.2;
   the fusion protein is any one of the following:

   A1) a protein whose amino acid sequence is SEQ ID No.1;
   A2) a protein having 80% or more identity with the protein shown in A1) and having the same function obtained by replacing and/or deleting and/or adding amino acid residues of the amino acid sequence shown in SEQ ID No.1;
   A3) a fusion protein having the same function obtained by attaching a tag to the N-terminus and/or C-terminus of A1) or A2).

4. The nucleic acid molecule according to any one of claims 1-3, wherein the nucleic acid molecule further comprises an IFNα gene and/or a P2A gene.

5. The nucleic acid molecule according to any one of claims 1-3, wherein the encoding gene of the chimeric antigen receptor is an encoding gene of a chimeric antigen receptor targeting GPC3.

6. The nucleic acid molecule according to claim 5, wherein the encoding gene of the chimeric antigen receptor targeting GPC3 is any one of the following:

C1) a nucleic acid molecule encoding a protein having an amino acid sequence of SEQ ID No.4;
C2) a DNA molecule having an encoding sequence of position 1-1473 of SEQ ID No.3;
C3) a DNA molecule having a nucleotide sequence of position 1-1473 of SEQ ID No.3.

7. The nucleic acid molecule according to claim 6, wherein the nucleic acid molecule is any one of the following:

D1) a DNA molecule having a nucleotide sequence of SEQ ID No.3;
D2) a DNA molecule having 70% or more identity with the DNA molecule shown in D1) and having the same function obtained by modifying the nucleotide sequence and/or replacing and/or deleting and/or adding one or more nucleotides shown in SEQ ID No.3.

8. Biomaterial, wherein the biomaterial is any one of the following:

E1) an expression cassette containing the nucleic acid molecule of any one of claims 1-7;
E2) a recombinant vector containing the nucleic acid molecule of any one of claims 1-7, or a recombinant vector containing the expression cassette of E1);
E3) a recombinant microorganism containing the nucleic acid molecule of any one of claims 1-7, or a recombinant microorganism containing the expression cassette of E1), or a recombinant microorganism containing the recombinant vector of E2);
E4) a recombinant cell containing the nucleic acid molecule of any one of claims 1-7, or a recombinant cell containing the expression cassette of E1), or a recombinant cell containing the recombinant vector of E2);
E5) the encoding gene of the chimeric switch receptor of claim 2 or 3;
E6) the chimeric switch receptor of claim 2;
E7) the fusion protein of claim 3.

9. The biomaterial according to claim 8, wherein the cell of E4) is a T cell, a NK cell, a γδ T cell, a NKT cell, a macrophage or a stem cell.

10. The nucleic acid molecule of any one of claims 1-7, and/or any of the following uses of the biomaterial of claim 8 or 9:

F1) use in the preparation of a drug for preventing or treating tumors;
F2) use in the preparation of a drug for preventing or treating tumors expressing the GPC3 antigen;
F3) use in regulating the immunosuppressive effect of the tumor microenvironment or in the preparation of a product regulating the immunosuppressive effect of the tumor microenvironment;
F4) use in the prevention or treatment of tumors;
F5) use in the prevention or treatment of tumors expressing the GPC3 antigen;
F6) use in the prevention or treatment of liver cancer, melanoma, Wilm's tumor, non-small cell lung cancer, ovarian clear cell carcinoma, squamous cell carcinoma, renal cell carcinoma, prostate cancer, colorectal cancer, hepatoblastoma or glioma.

11. A pharmaceutical composition, wherein the active ingredient of the pharmaceutical composition is a CAR cell containing or expressing any of the nucleic acid molecules of the present invention.

12. A method for preventing or treating tumors, wherein the method is to administer a drug containing the recombinant cell of claim 8 to a patient suffering from a tumor disease.

13. The method according to claim 12, wherein the recombinant cell is a GPC3 CAR-IC T cell, and the GPC3 CAR-IC T cell contains the DNA molecule shown in SEQ ID No.3.

14. The method according to claim 12 or 13, wherein the tumor is a tumor expressing the GPC3 antigen.

15. The method according to claim 14, wherein the tumor expressing the GPC3 antigen is liver cancer, melanoma, Wilm's tumor, non-small cell lung cancer, ovarian clear cell carcinoma, squamous cell carcinoma, renal cell carcinoma, prostate cancer, colorectal cancer, hepatoblastoma or glioma.

16. The method according to claim 15, wherein the liver cancer is hepatocellular carcinoma.

17. The method according to claim 15, wherein the squamous cell carcinoma is lung squamous cell carcinoma.

18. The method according to claim 15, wherein the melanoma is malignant melanoma.

19. The method of claim 15, wherein the glioma is glioblastoma.

Figure 1

Figure 2

Figure 3

Figure 4

Figure 5

Figure 6

Figure 7

# INTERNATIONAL SEARCH REPORT

| International application No. |
| --- |
| **PCT/CN2022/134772** |

| A. | CLASSIFICATION OF SUBJECT MATTER |
| --- | --- |

C12N15/62(2006.01)i; C07K19/00(2006.01)i; A61K39/00(2006.01)i; A61P35/00(2006.01)i; A61P37/02(2006.01)i

According to International Patent Classification (IPC) or to both national classification and IPC

| B. | FIELDS SEARCHED |
| --- | --- |

Minimum documentation searched (classification system followed by classification symbols)

C12N,C07K,A61K,A61P

Documentation searched other than minimum documentation to the extent that such documents are included in the fields searched

Electronic data base consulted during the international search (name of data base and, where practicable, search terms used)

CNTXT, DWPI, ENTXT, ENTXTC, VEN, WPABS, WPABSC, PUBMED, ISI WEB OF SCIENCE, GenBank, STN: 嵌合抗原受体, 嵌合转换开关受体, 嵌合转换受体, CAR, CSR, chimeric switch receptor, chimeric antigen receptor, 磷脂酰肌醇蛋白聚糖3, glypican-3, GPC3, IFN, P2A, TGF-β, 融合蛋白, fusion, CD27, PD-1, 癌症, 肝癌, SEQ ID NOs: 1-3

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| X | US 2022249563 A1 (NANJING LEGEND BIOTECH CO., LTD.) 11 August 2022 (2022-08-11)<br>claims 38-65 | 1, 4, 8-12, 15, 18-19 |
| X | WO 2022007665 A1 (HANGZHOU CHEETAH CELL THERAPEUTICS CO., LTD.) 13 January 2022 (2022-01-13)<br>claims 1-2, and description, paragraphs 111-118 | 1, 4, 8-12, 15, 18-19 |
| X | CN 113272318 A (KONG SEOG-KYOUNG) 17 August 2021 (2021-08-17)<br>description, paragraphs 18-53 | 1, 4, 8-12, 15, 18-19 |
| A | CN 114525260 A (SHENZHEN PRECODA LIFE SCIENCE CO., LTD.) 24 May 2022 (2022-05-24)<br>entire document | 1-19 |
| A | US 2021017249 A1 (JUNO THERAPEUTICS, INC. et al.) 21 January 2021 (2021-01-21)<br>entire document | 1-19 |

☑ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **10 August 2023** | **16 August 2023** |

| Name and mailing address of the ISA/CN | Authorized officer |
| --- | --- |
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
|---|
| **PCT/CN2022/134772** |

**C.    DOCUMENTS CONSIDERED TO BE RELEVANT**

| Category* | Citation of document, with indication, where appropriate, of the relevant passages | Relevant to claim No. |
|---|---|---|
| A | CN 114591443 A (FIRST AFFILIATED HOSPITAL OF WANNAN MEDICAL COLLEGE (YIJISHAN HOSPITAL OF WANNAN MEDICAL COLLEGE)) 07 June 2022 (2022-06-07) entire document | 1-19 |
| A | ZHANG, C. et al. "Chimeric Antigen Receptor- and Natural Killer Cell Receptor-engineered Innate Killer Cells in Cancer Immunotherapy" *Cellular & Molecular Immunology*, Vol. vol. 24, 15 July 2021 (2021-07-15), pp. 1-18 | 1-19 |
| A | MOHAMMED, S. et al. "Improving Chimeric Antigen Receptor-Modified T Cell Function by Reversing the Immunosuppressive Tumor Microenvironment of Pancreatic Cancer" *Molecular Therapy*, Vol. 25, No. (1), 31 January 2017 (2017-01-31), pp. 249-258 | 1-19 |
| A | 田永贵 等 (TIAN, Yonggui et al.). ""下一代"CAR T 细胞在实体瘤治疗中的研究进展 ("Next Generation" CAR T Cells in Solid Tumor Treatment)" *中国免疫学杂志 (Chinese Journal of Immunology)*, Vol. 36, No. (9), 31 December 2020 (2020-12-31), pp. 1025-1030 | 1-19 |
| A | QIN, L. et al. "Co-expression of a PD-L1-specific Chimeric Switch Receptor Augments the Efficacy and Persistence of CAR T Cells via the CD70-CD27 Axis" *Nature Communications*, Vol. vol. 13, 13 October 2022 (2022-10-13), Document number: 6051 | 1-19 |

Form PCT/ISA/210 (second sheet) (July 2022)

**INTERNATIONAL SEARCH REPORT**

International application No.

**PCT/CN2022/134772**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a.  ☑  forming part of the international application as filed.

    b.  ☐  furnished subsequent to the international filing date for the purposes of international search (Rule 13*ter*.1(a)),

        ☐  accompanied by a statement to the effect that the sequence listing does not go beyond the disclosure in the international application as filed.

2.  ☐  With regard to any nucleotide and/or amino acid sequence disclosed in the international application, this report has been established to the extent that a meaningful search could be carried out without a WIPO Standard ST.26 compliant sequence listing.

3.  Additional comments:

Form PCT/ISA/210 (continuation of first sheet) (July 2022)

## INTERNATIONAL SEARCH REPORT

| International application No. |
|---|
| **PCT/CN2022/134772** |

---

**Box No. II      Observations where certain claims were found unsearchable (Continuation of item 2 of first sheet)**

This international search report has not been established in respect of certain claims under Article 17(2)(a) for the following reasons:

1. ☑ Claims Nos.: **10 (in part), 12-19**
   because they relate to subject matter not required to be searched by this Authority, namely:

   Claims 10 (in part) and 12-19 relate to a method for treating a tumor, which falls within methods for treatment of diseases as defined in PCT Rule 39.1(iv). However, a search is stilled carried out on the basis of the pharmaceutical use thereof.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2022/134772**

| Patent document cited in search report | | | Publication date (day/month/year) | Patent family member(s) | | | Publication date (day/month/year) |
|---|---|---|---|---|---|---|---|
| US | 2022249563 | A1 | 11 August 2022 | JP | 2022542543 | A | 05 October 2022 |
| | | | | EP | 3999550 | A1 | 25 May 2022 |
| | | | | CA | 3146019 | A1 | 21 January 2021 |
| | | | | WO | 2021008610 | A1 | 21 January 2021 |
| | | | | AU | 2020314509 | A1 | 06 January 2022 |
| | | | | TW | 202116812 | A | 01 May 2021 |
| | | | | KR | 20220035367 | A | 22 March 2022 |
| WO | 2022007665 | A1 | 13 January 2022 | None | | | |
| CN | 113272318 | A | 17 August 2021 | US | 2022387503 | A1 | 08 December 2022 |
| | | | | BR | 112021004289 | A2 | 03 August 2021 |
| | | | | WO | 2020050667 | A1 | 12 March 2020 |
| | | | | CA | 3111978 | A1 | 12 March 2020 |
| | | | | AU | 2019336031 | A1 | 15 April 2021 |
| | | | | EP | 3848387 | A1 | 14 July 2021 |
| | | | | EP | 3848387 | A4 | 22 June 2022 |
| | | | | US | 2023055761 | A1 | 23 February 2023 |
| | | | | US | 2021252069 | A1 | 19 August 2021 |
| | | | | KR | 20210049916 | A | 06 May 2021 |
| | | | | JP | 2021536266 | A | 27 December 2021 |
| | | | | US | 2023056345 | A1 | 23 February 2023 |
| | | | | IL | 281232 | A | 29 April 2021 |
| CN | 114525260 | A | 24 May 2022 | None | | | |
| US | 2021017249 | A1 | 21 January 2021 | IL | 277702 | A | 30 November 2020 |
| | | | | JP | 2021520202 | A | 19 August 2021 |
| | | | | EP | 3775238 | A1 | 17 February 2021 |
| | | | | SG | 11202009313 | VA | 29 October 2020 |
| | | | | MA | 52656 | A | 17 February 2021 |
| | | | | CA | 3094468 | A1 | 10 October 2019 |
| | | | | WO | 2019195492 | A1 | 10 October 2019 |
| | | | | RU | 2020135966 | A | 06 May 2022 |
| | | | | KR | 20210029707 | A | 16 March 2021 |
| | | | | MX | 2020010459 | A | 20 January 2021 |
| | | | | AU | 2019247200 | A1 | 15 October 2020 |
| | | | | AU | 2019247200 | A2 | 09 June 2022 |
| | | | | BR | 112020020245 | A2 | 06 April 2021 |
| CN | 114591443 | A | 07 June 2022 | None | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **ENGELS B** ; **CAM H et al.** Retroviral Vectors for High-Level Transgene Expression in T Lymphocytes [J].. *Human Gene Therapy*, 2003, vol. 14 (12), 1155-1168